# EUROPEAN PATENT APPLICATION

(11) **EP 2 324 826 A1**
(43) Date of publication of application: **25.05.2011**
(21) Application number: 08808886.9
(22) Date of filing: 11.08.2008
(51) Int. Cl.: A61K 31/198, A23L 1/22, A23L 1/305, A61K 9/10, A61K 9/14, A61K 9/20, A61K 9/48, A61K 47/08, A61K 47/12, A61K 47/36, A61K 47/38, A61P 1/14, A61P 3/02, A61P 17/14, A61P 17/16, A61P 21/00, A61P 37/04, A61P 43/00

(54) **HYDROPHILIC AMINO ACID-CONTAINING PREPARATION HAVING IMPROVED TASTE**

(71) Applicant: Ajinomoto Co., Inc., Tokyo 104-8315 (JP)
(72) Inventor: TANAKA, Masayuki, Kawasaki-shi Kanagawa 210-8681 (JP); KITAZAWA, Manabu, Kawasaki-shi Kanagawa 210-8681 (JP); MAKINO, Chisato, Kawasaki-shi Kanagawa 210-8681 (JP); TAKANOSU, Kazuhiro, Kawasaki-shi Kanagawa 210-8681 (JP)
(74) Representative: Nicholls, Kathryn Margaret
(86) International application number: PCT/JP2008/064401
(87) International publication number: WO 2010/018614

(57) **Abstract**

It is intended to provide a hydrophilic amino acid-containing preparation wherein the taste of the hydrophilic amino acid is improved without damaging the appearance, flavor, storage stability and so on. It is further intended to provide a hydrophilic amino acid-containing preparation in which quick release of the hydrophilic amino acid from the preparation is assured, if necessary. It is furthermore intended to provide a hydrophilic amino acid-containing preparation **characterized in that** a solid agent containing a hydrophilic amino acid is coated with a coating agent comprising a flavoring agent and a water-soluble high molecular material.

## Description

### TECHNICAL FIELD

The present invention relates to a hydrophilic amino acid-containing preparation having an improved taste.

### BACKGROUND ART

Hydrophilic amino acids are widely used in pharmaceutical drugs, medical foods, health nutritious foods, health nutrition supplements, and the like for the purpose of disease treatment and prevention, maintenance and promotion of skin's beauty, hair stimulation and growth, anti-aging, nutrient enrichment and supplement, immune enhancement, fatigue recovery, muscle building, growth aids, and so forth. However, hydrophilic amino acids often have undesirable tastes or flavors such as bitterness, and are thus desired to have an improved taste.

To improve the taste, a sweetening agent, for example, sugars such as white sugar, dextrose, and fructose is generally used in combination. However, when sugars come into contact with an amino acid, what is called the Maillard reaction take places, deteriorating the appearance, flavor, storage stability, and the like.

As an example of a technique using an additive in combination other than sweetening agents, JP-A 2003-235512 describes a method in which a different amino acid from an amino acid having undesirable taste or flavor is used in combination. Nevertheless, the method has problems, in addition to that the amount used in the combination becomes large, that the pharmacological action exhibited by the different amino acid limits its use, and so forth.

As a technique to improve the taste in a relatively small amount, there is a method in which a coating agent is applied. However, coating on a preparation significantly influences releasing of a component from the preparation. This limits application of such a method to preparations from which immediate release of a component is essential.
[Patent Document 1] JP-A 2003-235512

### DISCLOSURE OF THE INVENTION

An object to be achieved by the present invention is to obtain a hydrophilic amino acid-containing preparation with a hydrophilic amino acid having an improved taste without deteriorating the appearance, flavor, storage stability, and the like. Furthermore, another object thereof is to obtain a hydrophilic amino acid-containing preparation which ensures immediate release of the hydrophilic amino acid from the preparation as necessary.

The present inventors have conducted various studies to achieve the above objects from the viewpoint of drug formation. As a result, the inventors have discovered that when a solid formulation containing a hydrophilic amino acid is coated with a coating agent formed from a corrigent and a water-soluble polymer substance, a hydrophilic amino acid-containing preparation with the hydrophilic amino acid having an improved taste is obtained without deteriorating the appearance, flavor, storage stability, and the like. Furthermore, the inventors have discovered that, by adjusting the ratio between the coating agent and the solid formulation containing a hydrophilic amino acid, it is possible to ensure immediate release of the hydrophilic amino acid from the preparation. Based on such findings, the present inventors have further conducted studies and thus completed the present invention. The present invention includes the following in the list.
(1) A hydrophilic amino acid-containing preparation characterized in that a solid formulation containing a hydrophilic amino acid is coated with a coating agent containing a corrigent and a water-soluble polymer substance, and the hydrophilic amino acid is in a form selected from the group consisting of a free form, a salt, a solvate, and a mixture thereof.
(2) The hydrophilic amino acid-containing preparation according to the above-described (1), characterized in that the hydrophilic amino acid is selected from the group consisting of arginine, arginine salts, and mixtures of these.
(3) The hydrophilic amino acid-containing preparation according to the above-described (1), characterized in that the hydrophilic amino acid is selected from the group consisting of lysine, lysine salts, histidine, histidine salts, and mixtures of these.
(4) The hydrophilic amino acid-containing preparation according to any one of the above-described (1) to (3), characterized in that the solid formulation containing the hydrophilic amino acid further contains, in addition to the hydrophilic amino acid, an amino acid other than the hydrophilic amino acid, and the amino acid other than the hydrophilic amino acid is in a form selected from the group consisting of a free form, a salt, a solvate, and a mixture thereof.
(5) The hydrophilic amino acid-containing preparation according to any one of the above-described (1) to (4), characterized in that the corrigent is selected from the group consisting of aspartame, saccharin, sodium saccharin, glycyrrhizic acid, glycyrrhizates, acesulfame K, mannitol, erythritol, sorbitol, xylitol, trehalose, cacao powder, menthol, thaumatin, stevia, sucralose, maltitol, refined white sugar, white sugar, dextrose, fructose, and mixtures thereof.
(6) The hydrophilic amino acid-containing preparation according to any one of the above-described (1) to (5), characterized in that the water-soluble polymer substance is selected from the group consisting of hydroxypropyl cellulose, hydroxypropylmethyl cellulose, sodium carboxymethyl cellulose, methyl cellulose, gum arabic, polyvinylpyrrolidone (povidone), dextrin, sodium alginate, casein, and mixtures thereof.
(7) The hydrophilic amino acid-containing preparation according to any one of the above-described (1) to (4), characterized in that the corrigent is mannitol, and the water-soluble polymer substance is hydroxypropyl cellulose.
(8) The hydrophilic amino acid-containing preparation according to any one of the above-described (1) to (7), characterized in that the coating agent further contains, in addition to the corrigent and the water-soluble polymer substance, one or more kinds selected from souring agents, plasticizers and lubricants.
(9) The hydrophilic amino acid-containing preparation according to the above-described (8), characterized in that the souring agent is selected from the group consisting of citric acid, malic acid, acetic acid, tartaric acid, ascorbic acid, glutamic acid, glutamates, lactic acid, succinic acid, maleic acid, malonic acid, and mixtures thereof.
(10) The hydrophilic amino acid-containing preparation according to the above-described (8) or (9), characterized in that the plasticizer is selected from the group consisting of macrogol, propylene glycol, triethyl citrate, castor oil, triacetin, polysorbate nonionic surfactants, sorbitan ester nonionic surfactants, and mixtures thereof.
(11) The hydrophilic amino acid-containing preparation according to any one of the above-described (8) to (10), characterized in that the lubricant is selected from the group consisting of talc, stearic acid, stearates, waxes, wheat starches, and mixtures thereof.
(12) A solid preparation selected from a powder, a fine granule, a granule, a dry syrup, a pill, a tablet, a chewable, a capsule, and a microcapsule which are made of the hydrophilic amino acid-containing preparation according to any one of the above-described (1) to (11).
(13) A hydrophilic amino acid-containing granule characterized by being made of the hydrophilic amino acid-containing preparation according to any one of the above-described (1) to (11), the granule characterized in that a ratio of the solid formulation containing the hydrophilic amino acid to the coating agent is 100/1 to 100/100 in weight ratio.
(14) A hydrophilic amino acid-containing granule characterized by being made of the hydrophilic amino acid-containing preparation according to any one of the above-described (1) to (11), the granule further characterized in that the corrigent is mannitol, the water-soluble polymer substance is hydroxypropyl cellulose, and a ratio of the solid formulation containing the hydrophilic amino acid to the coating agent is 100/5 to 100/40 in weight ratio.
(15) A hydrophilic amino acid-containing tablet characterized by being made of the hydrophilic amino acid-containing preparation according to any one of the above-described (1) to (11), the tablet characterized in that, a ratio of the solid formulation containing the hydrophilic amino acid to the coating agent is 100/0.01 to 100/50 in weight ratio.
(16) A hydrophilic amino acid-containing tablet characterized by being made of the hydrophilic amino acid-containing preparation according to any one of the above-described (1) to (11), the tablet further characterized in that the corrigent is mannitol, the water-soluble polymer substance is hydroxypropyl cellulose, and a ratio of the solid formulation containing the hydrophilic amino acid to the coating agent is 100/0.1 to 100/20 in weight ratio.

The present invention provides a hydrophilic amino acid-containing preparation with a hydrophilic amino acid having improved taste without deteriorating the appearance, flavor, storage stability, and the like. Furthermore, provided is a hydrophilic amino acid-containing preparation which ensures immediate release of the hydrophilic amino acid from the preparation as necessary. The hydrophilic amino acid-containing preparation of the present invention is useful when a hydrophilic amino acid is used as pharmaceutical drugs, medical foods, health nutritious foods, health nutrition supplements, and the like for the purpose of disease treatment and prevention, maintenance and promotion of skin's beauty, hair stimulation and growth, anti-aging, nutrient enrichment and supplement, immune enhancement, fatigue recovery, muscle building, growth aids, and so forth.

### BEST MODE FOR CARRYING OUT THE INVENTION

Hereinafter, an embodiment of the present invention will be described.

In a hydrophilic amino acid-containing preparation of the present invention, a hydrophilic amino acid used in a solid formulation for containing the hydrophilic amino acid is not particularly limited, as long as a side chain of the amino acid is hydrophilic. Examples thereof include amino acids each containing a functional group such as a hydroxyl group, an amide group, a carboxyl group, an amino group, a guanidino group, an imidazole group, or the like at the side chain of the amino acid as described in "An Introduction to Protein Science for Bio-Science" (written by Fumio Arisaka, SHOKABO Publishing Co., Ltd., 2006, pp. 30 to 34). Specific examples thereof include serine, threonine, aspartic acid, glutamic acid, asparagine, glutamine, arginine, lysine, histidine, and the like. These are, for example, amino acids having a hydropathy index of 1.5 or lower, preferably -3.0 or lower, and more preferably -8.0 or lower measured using the hydrophobicity scale according to D. A. Engelman, T. A. Steitz, and A. Goldman, among the hydrophobicity scales as described in "Introduction to Protein Structure" (Carl Branden (author), John Tooze (author), Yukiteru Katsube et al. (translators), Kyoikusha, 1992, pp. 210 to 211). In a case where an asymmetric carbon atom is present, the amino acid may be either optically active or racemic.

The hydrophilic amino acid used in the present invention may be in any form of a free form, a salt, and a solvate thereof, or may be a mixture thereof.

Examples of the salt include inorganic acid salts such as hydrochloric acid salts, sulfuric acid salts, carbonic acid salts, and phosphoric acid salts, organic acid salts such as acetic acid salts, tartaric acid salts, citric acid salts, p-toluenesulfonic acid salts, glycolic acid salts, malic acid salts, lactic acid salts, succinic acid salts, maleic acid salts, fumaric acid salts, malonic acid salts, gluconic acid salts, saccharic acid salts, benzoic acid salts, fatty acid salts, and pyroglutamic acid salts, acidic amino acid salts such as aspartic acid salts, and glutamic acid salts, metal salts such as sodium salts, potassium salts, magnesium salts, calcium salts, zinc salts, and copper salts, salts of inorganic bases such as ammonia, salts of amines such as monoethanolamine, diethanolamine, and triethanolamine, salts of basic amino acids such as lysine, arginine, ornithine, and histidine, and the like. These salts may be either one kind alone or in combination of two or more kinds. These salts may be used in the form of amino acid salts, or the amino acids in the free form and the salts may be used separately from each other to form amino acid salts in the preparation.

Examples of a solvent for forming the solvate include water, methanol, ethanol, isopropanol, ethyl acetate, isopropyl acetate, and the like. The solvent for forming the solvate may be either one kind alone or in combination of two or more kinds.

The hydrophilic amino acid used in the present invention is not particularly limited, as long as the amino acid is hydrophilic and has some taste.

Examples of such a taste that the hydrophilic amino acid has include bitterness, sourness, saltiness, salty pungency, sweetness different from that of sugar, and other tastes. These tastes may be either one kind alone or in combination of two or more kinds. Examples thereof include sweetness that D-serine, L-threonine, D-threonine, D-asparagine, D-glutamine, and D-histidine have and different from that of sugar; tastes in combination of bitterness, sourness, and sweetness that L-serine has, the sweetness being different from that of sugar; sourness that L-aspartic acid, D-aspartic acid, L-glutamic acid, and D-glutamic acid have; tastes in combination of bitterness and sourness that L-asparagine has; tastes in combination of saltiness and bitterness that L-glutamine has; bitterness that L-arginine and L-histidine have; tastes in combination of bitterness and sweetness that L-arginine hydrochloride, D-arginine, L-lysine, and D-lysine have, the sweetness being different from that of sugar; salty pungency that L-lysine hydrochloride and L-histidine hydrochloride have; and other tastes.

It is particularly important to improve the tastes of, among the hydrophilic amino acids, arginine and arginine salts that particularly influence not only bitterness but also aftertaste for the elderly who have reduced swallowing ability, so that a preparation is likely to stay in the oral cavity. In addition, it is also said to be an important preparation property that a preparation being taken can be rapidly mixed well with saliva so as to be swallowed without aggregation in the oral cavity.

Among the hydrophilic amino acids, lysine, lysine salts, histidine and histidine salts have strong tastes. Accordingly, when preparations containing these are taken over an extended period, the improvement in the tastes thereof is particularly important.

In the hydrophilic amino acid-containing preparation of the present invention, the content of the hydrophilic amino acid is not particularly limited. However, to prepare the preparation, the content is preferably 10 to 100% by weight in the solid formulation containing the hydrophilic amino acid.

In the hydrophilic amino acid-containing preparation of the present invention, in addition to the hydrophilic amino acid, an amino acid other than the hydrophilic amino acid may be used in the solid formulation containing the hydrophilic amino acid. Examples of the amino acid other than the hydrophilic amino acid include alanine, isoleucine, leucine, valine, cysteine, cystine, methionine, phenylalanine, tryptophan, and the like. In a case where an asymmetric carbon atom is present, the amino acid other than the hydrophilic amino acid may be either optically active or racemic similarly to the hydrophilic amino acid. Furthermore, the amino acid other than the hydrophilic amino acid may be in any form of a free form, a salt, and a solvate thereof, or may be a mixture thereof.

In the hydrophilic amino acid-containing preparation of the present invention, besides the hydrophilic amino acid, various additives that are used in pharmaceutical drug preparations, foods, and the like can be used in the solid formulation containing the hydrophilic amino acid to prepare the preparation. Examples of such additives include excipients such as crystalline cellulose, various starches, calcium hydrogen phosphate, light anhydrous silicic acid, titanium oxide, magnesium aluminometasilicate, polyethylene glycol, and mannitol, binders such as crystalline cellulose, methyl cellulose, ethyl cellulose, hydroxypropyl cellulose, hydroxypropylmethyl cellulose, carmellose sodium, various starches, gum arabic, agar, gelatins, tragacanth, sodium alginate, polyvinylpyrrolidone (povidone), and polyvinyl alcohol, disintegrators such as crystalline cellulose, low-substituted hydroxypropyl cellulose, carmellose (carboxymethyl cellulose), carmellose calcium, croscarmellose sodium, carboxymethylethyl cellulose, crospovidone, carboxymethyl starch sodium, hydroxypropyl starch, and partially pregelatinized starches, lubricants such as talc, stearic acid, stearates, waxes, and wheat starches, colorants such as tar dyes and titanium oxide, odor modifiers such as citric acid, adipic acid, ascorbic acid, menthol, fennel oil, cinnamon oil, ethyl vanillin, orange oil, and lemon oil, surfactants such as monostearic acid, glycerin, polysorbates (for example, polysorbate 60, polysorbate 65, polysorbate 80, and the like), sodium lauryl sulfate, and sucrose fatty acid esters, and the like.

Furthermore, in the hydrophilic amino acid-containing preparation of the present invention, a different corrigent from the corrigent contained in a coating agent covering the solid formulation containing the hydrophilic amino acid can be contained in the solid formulation containing the hydrophilic amino acid. Examples of the corrigent which is different from the corrigent contained in the coating agent covering the solid formulation containing the hydrophilic amino acid, and which can be contained in the solid formulation containing the hydrophilic amino acid include aspartame, saccharin, sodium saccharin, glycyrrhizic acid, monoammonium glycyrrhizate, diammonium glycyrrhizate, dipotassium glycyrrhizate, disodium glycyrrhizate, trisodium glycyrrhizate, acesulfame K, mannitol, erythritol, sorbitol, xylitol, trehalose, cacao powder, menthol, thaumatin, stevia, sucralose, maltitol, and the like.

Still furthermore, in the hydrophilic amino acid-containing preparation of the present invention, a different coating agent from the coating agent covering the solid formulation containing the hydrophilic amino acid can be contained in the solid formulation containing the hydrophilic amino acid. Examples of the coating agent which is different from the coating agent covering the solid formulation containing the hydrophilic amino acid, and which can be contained in the solid formulation containing the hydrophilic amino acid include methyl cellulose, ethyl cellulose, hydroxypropyl cellulose, hydroxypropylmethyl cellulose, hydroxypropylmethyl cellulose phthalate, polyvinylpyrrolidone (povidone), sodium carboxymethyl cellulose, gum arabic, dextrin, sodium alginate, casein, mannitol, polyvinyl acetal diethylaminoacetate, aminoalkyl methacrylate copolymers, polyvinyl acetate phthalate, polyethylene glycol (for example, macrogol and the like), and the like.

In the hydrophilic amino acid-containing preparation of the present invention, the solid formulation containing the hydrophilic amino acid may be a commercially available hydrophilic amino acid-containing preparation that has some taste. Examples thereof include Argi-U (registered trademark) granule (Ajinomoto Pharma Co., Ltd.), Amiyu (registered trademark) granule (Ajinomoto Pharma Co., Ltd.), and the like.

In the hydrophilic amino acid-containing preparation of the present invention, the solid formulation containing the hydrophilic amino acid can be produced by using a extrusion granulator, a fluidized bed granulator, a high speed mixing granulator, a planetary mixer, a dry pressing granulator, a crush granulator, an oscillating granulator, a spray drying granulator, a coating granulator, and the like.

In the hydrophilic amino acid-containing preparation of the present invention, the coating agent covering the solid formulation containing the hydrophilic amino acid contains a corrigent and a water-soluble polymer substance.

The corrigent used in the coating agent can be selected from corrigents that are used in pharmaceutical drug preparations, foods, and the like. Preferable examples thereof include aspartame, saccharin, sodium saccharin, glycyrrhizic acid, monoammonium glycyrrhizate, diammonium glycyrrhizate, dipotassium glycyrrhizate, disodium glycyrrhizate, trisodium glycyrrhizate, acesulfame K, mannitol, erythritol, sorbitol, xylitol, trehalose, cacao powder, menthol, thaumatin, stevia, sucralose, maltitol, refined white sugar, white sugar, dextrose, fructose, other sugars, and the like. More preferable examples thereof include aspartame, sodium saccharin, and mannitol. These may be either one kind alone or in combination of two or more kinds.

The water-soluble polymer substance used in the coating agent can be selected from coating agents, binders, and the like that are used in pharmaceutical drug preparations, foods, and the like. Preferable examples thereof include hydroxypropyl cellulose, hydroxypropylmethyl cellulose, sodium carboxymethyl cellulose, methyl cellulose, gum arabic, polyvinylpyrrolidone (povidone), dextrin, sodium alginate, casein, and the like. More preferable examples include hydroxypropyl cellulose and polyvinylpyrrolidone (povidone). These may be either one kind alone or in combination of two or more kinds.

In the hydrophilic amino acid-containing preparation of the present invention, the content of the coating agent covering the solid formulation containing the hydrophilic amino acid, in terms of the weight ratio of the solid formulation to the coating agent, is selected within the range from 100/0.001 to 100/200. If the weight ratio of the solid formulation to the coating agent is below 100/0.001, the effect of improving the taste of the hydrophilic amino acid is not sufficiently demonstrated in some cases. If the weight ratio of the solid formulation to the coating agent exceeds 100/200, the time required for coating in producing the preparation becomes long, which is not preferable.

In a case where the hydrophilic amino acid-containing preparation of the present invention is a granule, the content of the coating agent on the solid formulation containing the hydrophilic amino acid, in terms of the weight ratio of the solid formulation to the coating agent, is preferably selected within the range from 100/1 to 100/100. Moreover, in a case where the hydrophilic amino acid-containing preparation is a granule, and besides, the corrigent used in the coating agent is mannitol and the water-soluble polymer substance is hydroxypropyl cellulose, the weight ratio of the solid formulation to the coating agent is preferably selected within the range from 100/5 to 100/40.

In a case where the hydrophilic amino acid-containing preparation of the present invention is a tablet, the content of the coating agent on the solid formulation containing the hydrophilic amino acid, in terms of the weight ratio of the solid formulation to the coating agent, is preferably selected within the range from 100/0.01 to 100/50. Moreover, in a case where the hydrophilic amino acid-containing preparation is a tablet, and besides, the corrigent used in the coating agent is mannitol and the water-soluble polymer substance is hydroxypropyl cellulose, the weight ratio of the solid formulation to the coating agent is preferably selected within the range from 100/0.1 to 100/20.

In the hydrophilic amino acid-containing preparation of the present invention, the weight ratio of the corrigent to the water-soluble polymer substance in the coating agent covering the solid formulation containing the hydrophilic amino acid is not particularly limited. However, to prepare the preparation, the weight ratio is preferably selected within the range from 100/1 to 1/100.

In the hydrophilic amino acid-containing preparation of the present invention, the coating agent covering the solid formulation containing the hydrophilic amino acid can further contain, in addition to the corrigent and the water-soluble polymer substance, one or more kinds selected from souring agents, plasticizers and lubricants.

The souring agent used in the coating agent can be selected from souring agents that are used in pharmaceutical drug preparations, foods, and the like. Preferable examples thereof include citric acid, malic acid, acetic acid, tartaric acid, ascorbic acid, glutamic acid, glutamates, lactic acid, succinic acid, maleic acid, malonic acid, and the like. These may be either one kind alone or in combination of two or more kinds.

The plasticizer used in the coating agent can be selected from plasticizers that are used in pharmaceutical drug preparations, foods, and the like. Preferable examples thereof include macrogol, propylene glycol, triethyl citrate, castor oil, triacetin, polysorbate nonionic surfactants such as polysorbate 80, sorbitan ester nonionic surfactants such as sorbitan fatty acid esters. These may be either one kind alone or in combination of two or more kinds.

The lubricant used in the coating agent can be selected from lubricants that are used in pharmaceutical drug preparations, foods, and the like. Preferable examples thereof include talc, stearic acid, stearates, waxes, wheat starches, and the like. These may be either one kind alone or in combination of two or more kinds.

In the hydrophilic amino acid-containing preparation of the present invention, besides the corrigent, the water-soluble polymer substance, the souring agent, the plasticizer and the lubricant, various additives that are used in pharmaceutical drug preparations, foods, and the like can be used in the coating agent covering the solid formulation containing the hydrophilic amino acid. Examples of such additives include colorants such as tar dyes and titanium oxide; odor modifiers such as citric acid, adipic acid, ascorbic acid, menthol, fennel oil, cinnamon oil, ethyl vanillin, orange oil, and lemon oil; surfactants such as monostearic acid, glycerin, polysorbates (for example, polysorbate 60, polysorbate 65, polysorbate 80, and the like), sodium lauryl sulfate, and sucrose fatty acid esters; coating agents other than water-soluble polymer substances, such as ethyl cellulose, hydroxypropylmethyl cellulose phthalate, polyvinyl acetal diethylaminoacetate, aminoalkyl methacrylate copolymers, polyvinyl acetate phthalate, and polyethylene glycol (for example, macrogol and the like); additives for preventing aggregation and adhesion of the preparation; additives for controlling and adjusting elution of the hydrophilic amino acid from the preparation so that the hydrophilic amino acid can be eluted in an accelerated, delayed, temporal or local manner; additives for further promoting the taste improvement; additives for promoting the stability, physiological characteristics, and the like of the preparation; and the like.

One coating layer or two or more coating layers may be formed by the coating in the present invention. When two or more coating layers are formed, the combination of the corrigent with the water-soluble polymer substance may be the same in all the layers, or the same only in some layers, or different among the layers. Even when the combination of the corrigent with the water-soluble polymer substance is the same, the coating layers can be considered different from each other, for example, in the cases where the coating layers are different in physical and chemical properties such as coating density; the coating layers are different in the weight ratio between the corrigent and the water-soluble polymer substance; the coating layers are different in composition other than the corrigent and the water-soluble polymer substance; and another coating layer having a different combination is present between the layers having the same combination.

When two or more coating layers are formed, the corrigent and the water-soluble polymer substance may be contained in different layers respectively. For example, suppose a case where there are two coating layers: an inner layer that is in direct contact with the solid formulation containing the hydrophilic amino acid; and an outer layer covering the inner layer. In this case, the inner layer may contain no corrigent but contain the water-soluble polymer substance, while the outer layer may contain the corrigent but contain no water-soluble polymer substance.

When two or more coating layers are formed, it is possible to form coating layers: one which contains both or one of the corrigent and water-soluble polymer substance; and one which does not contain any of the corrigent and the water-soluble polymer substance. The coating layer which does not contain any of the corrigent and the water-soluble polymer substance may be a gastric coating layer, an enteric coating layer, a coating layer dissolvable locally at other sites, a sustained-release coating layer, a coating layer, other than sustained-release coating layers, dissolvable in a time-dependent manner, and the like.

When two or more coating layers are formed, sugars such as refined white sugar, white sugar, dextrose, and fructose can be contained as the corrigent in a coating layer other than the coating layer that is in direct contact with the solid formulation containing the hydrophilic amino acid without causing the Maillard reaction.

In the hydrophilic amino acid-containing preparation of the present invention, the thickness of the coating layer covering the solid formulation containing the hydrophilic amino acid may be either uniform or non-uniform. Moreover, the coating film on the solid formulation containing the hydrophilic amino acid does not need to be complete, and part of the surface of the solid formulation does not necessarily have to be coated therewith.

For the hydrophilic amino acid-containing preparation of the present invention, a method for coating the solid formulation containing the hydrophilic amino acid may be a film coating method, a powder coating method, and the like. Preferable is a film coating method. In forming the coating film by the film coating method, a liquid formulation containing the coating agent is sprayed on the solid formulation containing the hydrophilic amino acid to form the coating layer on the surface of the solid formulation; thereby, the hydrophilic amino acid-containing preparation can be obtained. In forming the coating film by the powder coating method, while a liquid formulation containing no coating agent is sprayed on the solid formulation containing the hydrophilic amino acid, the coating agent is dispersed thereon to make the coating agent adhere on the surface of the solid formulation; thereby, the hydrophilic amino acid-containing preparation can be obtained.

Examples of equipment used for the coating in the present invention include fluidized bed coating equipment (for example, FLO series: Freund Corporation), fluidized bed coating equipment equipped with a Wurster column (for example, GPCG series: Glatt GmbH), centrifugal fluidizing coating equipment (for example, CF granulator: Freund Corporation), rolling fluidized bed coating equipment (for example, Multiplex: Powrex Corporation, New/Marumerizer: Fuji Paudal Co., Ltd. , AGGLOMASTER: Hosokawa Micron Corporation, SPIR-A-FLOW: Freund Corporation), improved fluidized bed coating equipment equipped with a Wurster column (for example, Multiplex: Powrex Corporation), pan coating equipment (for example, AQUA COATER, HI-COATER: both are from Freund Corporation), and the like.

In the hydrophilic amino acid-containing preparation of the present invention, after the solid formulation containing the hydrophilic amino acid is coated, normally it is dried, and the content of water and/or the solvent other than water in the preparation is adjusted adequately for the preparation. Normally, the adjustment is made such that the content should become equal to the content of water and/or the solvent other than water in the solid formulation containing the hydrophilic amino acid before the coating.

In the hydrophilic amino acid-containing preparation of the present invention, various additives that are used in pharmaceutical drug preparations, foods, and the like may be caused to adhere to the surface of the preparation. Examples of the additives caused to adhere to the surface of the preparation include souring agents such as citric acid, malic acid, acetic acid, tartaric acid, ascorbic acid, glutamic acid, glutamates, lactic acid, succinic acid, maleic acid, and malonic acid; plasticizers such as macrogol, propylene glycol, triethyl citrate, castor oil, triacetin, polysorbate nonionic surfactants such as polysorbate 80, and sorbitan fatty acid esters; plasticizers such as sorbitan ester nonionic surfactants, lubricants such as talc, stearic acid, stearates, waxes, and wheat starches; colorants such as tar dyes and titanium oxide; corrigents such as aspartame, saccharin, sodium saccharin, glycyrrhizic acid, monoammonium glycyrrhizate, diammonium glycyrrhizate, dipotassium glycyrrhizate, disodium glycyrrhizate, trisodium glycyrrhizate, acesulfame K, mannitol, erythritol, sorbitol, xylitol, trehalose, cacao powder, menthol, thaumatin, and sugars such as sucrose; odor modifiers such as citric acid, adipic acid, ascorbic acid, menthol, fennel oil, cinnamon oil, ethyl vanillin, orange oil, and lemon oil, surfactants such as monostearic acid, glycerin, polysorbates (for example, polysorbate 60, polysorbate 65, polysorbate 80, and the like), sodium lauryl sulfate, and sucrose fatty acid esters; additives for preventing aggregation and adhesion of the preparation; additives for controlling and adjusting elution of the hydrophilic amino acid from the preparation so that the hydrophilic amino acid can be eluted in an accelerated, delayed, temporal or local manner; additives for further promoting the taste improvement; additives for promoting the stability, physiological characteristics, and the like of the preparation; and the like.

The hydrophilic amino acid-containing preparation of the present invention can be in any preparation form such as a powder, a fine granule, a granule, a dry syrup, a pill, a tablet, a chewable, a capsule, and a microcapsule. Above all, a granule is preferable. Particularly preferable is a granule specified in the Japanese Pharmacopeia Fifteenth Edition (a preparation whose granules all pass through a No. 10 (1700 µm) sieve, not more than 5% of the total granules remain on a No. 12 (1400 µm) sieve, and not more than 15% of the total granules pass through a No. 42 (355 µm) sieve, when the particle size test described in the Japanese Pharmacopeia Fifteenth Edition is performed on the preparation). More preferable is a granule whose particle size distribution is further such that not more than 30% of granules thereof pass through a 500 µm sieve. Further preferable is a granule whose particle size distribution is further such that not more than 10% of granules thereof pass through a 500 µm sieve.

### Examples

Hereinafter, the present invention will be described in more details by use of Examples. However, the scope of the present invention is not limited thereto.

### Production Example 1 Coated Granule

Argi-U (registered trademark) granule (Ajinomoto Pharma Co., Ltd.) was film coated in accordance with the preparation formulations shown in Table 1 by using fluidized bed granule coating equipment (FLO-1 or FLO-5: Freund Corporation). After drying in the same equipment and sieving, various coated granules were obtained.

### [Table 1]

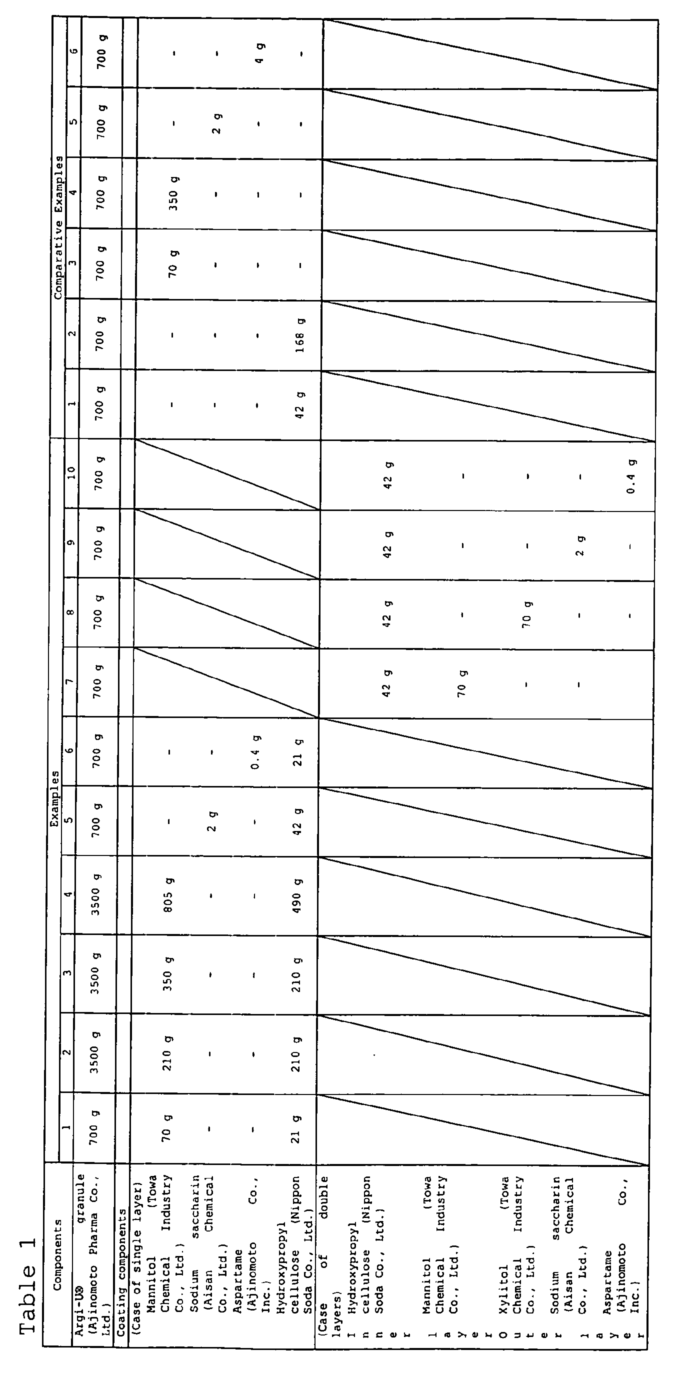

### Production Example 2 Coated Tablet

In accordance with the preparation formulations shown in Table 2, Argi-U (registered trademark) granule (Ajinomoto Pharma Co., Ltd.), carmellose calcium, and magnesium stearate were manually mixed, and then tablets were formed by using a rotary tablet press (HT-AP15SS-II: HATA IRON WORKS CO., LTD.). In this manner, uncoated tablets were obtained. The uncoated tablets thus obtained were film coated by using a tablet coating equipment (HI-COATER MINI: Freund Corporation). After drying with the same equipment, coated tablets were obtained.

### [Table 2]

**Table 2**

| Components | | Examples | | |
|---|---|---|---|---|
| | | 11 | 12 | 13 |
| Uncoated | Argi-U® | | | |
| tablet | granule | | | |
| components | (Ajinomoto Pharma Co., | 300 g | 300 g | 300 g |
| | Ltd.) | | | |
| | Carmellose | | | |
| | calcium | | | |
| | (Gotoku Chemical Company Ltd.) | 9.0 g | 9.0 g | 9.0 g |
| | Magnesium | | | |
| | stearate | | | |
| | (Taihei Chemical Industrial Co., Ltd.) | 1.5 g | 1.5 g | 1.5 g |
| Coating | Mannitol | | | |
| components | (Towa Chemical Industry Co., Ltd.) | 8.0 g | 16.0 g | 32.0 g |
| | Hydroxypropyl cellulose | | | |
| | cellulose Soda (Nippon Soda Co., Ltd.) | 4.8 g | 9.6 g | 19.2 g |

### Test Example 1 Sensory Evaluation of Taste

The coated granules obtained in Production Example 1 or the coated tablets obtained in Production Example 2 were taken by 2 to 5 panelists to perform a sensory evaluation of the taste. The evaluation criteria are as shown next. o: the taste (bitterness) is not unpleasant at all, Δ: the taste (bitterness) is little unpleasant, and ×: the taste (bitterness) is unpleasant.

### [Table 3]

**Table 3**

| | Sensory evaluation |
|---|---|
| Coated granule of Example 1 | ○ |
| Coated granule of Example 2 | ○ |
| Coated granule of Example 3 | ○ |
| Coated granule of Example 4 | ○ |
| Coated granule of Example 5 | ○ |
| Coated granule of Example 6 | ○ |
| Coated granule of Example 7 | ○ |
| Coated granule of Example 8 | ○ |
| Coated granule of Example 9 | ○ |
| Coated granule of Example 10 | ○ |
| Coated granule of | × |
| Comparative Example 1 | |
| Coated granule of | Δ |
| Comparative Example 2 | |
| Coated granule of | × |
| Comparative Example 3 | |
| Coated granule of | Δ |
| Comparative Example 4 | |
| Coated granule of | × |
| Comparative Example 5 | |
| Coated granule of | Δ |
| Comparative Example 6 | |
| Coated tablet of Example 11 | ○ |
| Coated tablet of Example 12 | ○ |
| Coated tablet of Example 13 | ○ |

As shown in Table 3, the products of the present invention all had sufficiently satisfactory taste improvement.

Even with arginine that particularly influences not only bitterness but also aftertaste among hydrophilic amino acids, the products of the present invention had an improved taste, and furthermore excellent ease of ingestion was obtained with little aggregation in the oral cavity. Thus, the products of the present invention can be said to be a quite favorable preparation for the elderly who have reduced swallowing ability and secrete a small amount of saliva.

### Test Example 2 Storage Stability Test

After the coated granules obtained in Production Example 1 were placed in a storage at 50°C for 2 weeks, the appearance was evaluated. The evaluation criteria are as shown next. ○: stable, and ×: unstable (coloring due to the storing was noticeable, the increase in degradation products exceeding 1%, or the like).

### [Table 4]

**Table 4**

| | Storage stability |
|---|---|
| Coated granule of Example 1 | ○ |
| Coated granule of Example 2 | ○ |
| Coated granule of Example 3 | ○ |
| Coated granule of Example 4 | ○ |
| Coated granule of Example 5 | ○ |
| Coated granule of Example 6 | ○ |
| Coated granule of Example 7 | ○ |
| Coated granule of Example 8 | ○ |
| Coated granule of Example 9 | ○ |
| Coated granule of Example 10 | ○ |

As shown in Table 4, the products of the present invention were all favorable in storage stability.

### Test Example 3 Dissolution Test

According to the paddle method described in the Japanese Pharmacopeia Fifteenth Edition or a method in conformity to this, the dissolution test was performed on each coated granule preparation obtained in Production Example 1. Five minutes after the test was started, the amount of arginine in the dissolution liquid was quantified, and the dissolution ratio was calculated. The evaluation criteria are as shown next. ○: immediate releasing is retained (dissolution ratio of 85% or higher), and x: immediate releasing is not retained (dissolution ratio of lower than 85%).

### [Table 5]

**Table 5**

| | Immediate release |
|---|---|
| Coated granule of Example 1 | ○ |
| Coated granule of Example 2 | ○ |
| Coated granule of Example 3 | ○ |
| Coated granule of Example 4 | ○ |
| Coated granule of Example 5 | ○ |
| Coated granule of Example 6 | ○ |
| Coated granule of Example 7 | ○ |
| Coated granule of Example 8 | ○ |
| Coated granule of Example 9 | ○ |
| Coated granule of Example 10 | ○ |

As shown in Table 5, the products of the present invention all had the immediate releasing retained, and the dissolution ratio was not lowered due to the coating.

### Production Example 2 Coated Granule

Amino acids were pulverized by using a sample mill (SAM-0: Nara Machinery Co., Ltd.) and a co-mill (QC-197S: Powrex Corporation) as necessary. Then, in accordance with the preparation formulations shown in Table 6, the amino acids were kneaded by using a stirring granulator FS-10: Freund Corporation), and granulated by using an extrusion granulator (EXD-60: Dalton Corporation). As necessary, the resultant was uniformly granulated by using a marumerizer (Q-230: Dalton Corporation). Thereafter, the granules were dried by using fluidized bed granule coating equipment (FLO-5: Freund Corporation), and uniformly granulated by using a speed mill (N10: Okada Seiko Co., Ltd.) as necessary. Uncoated granules thus produced or already-produced uncoated granules were film coated using fluidized bed granule coating equipment (FLO-1 or FLO-5: Freund Corporation). After drying in the same equipment and sieving, various coated granules were obtained.

### [Table 6]

**Table 6**

| Components | | Examples | | | | | |
|---|---|---|---|---|---|---|---|
| | | 14 | 15 | 16 | 17 | 18 | 19 |
| Uncoated granule producing step ingredients (g) | Argi-U® granule (Ajinomoto Pharma Co., Ltd.) | - | - | - | - | - | 300 |
| | Amiyu® granule (Ajinomoto Pharma Co., Ltd.) | 300 | - | - | - | - | - |
| | L-arginine L-glutamate hydrate (Ajinomoto Co., Inc.) | - | 1500 | - | - | - | - |
| | L-glutamine (Ajinomoto Co., Inc.) | - | - | 2000 | - | - | - |
| | L-lysine hydrochloride (Ajinomoto Co., Inc.) | - | - | - | 800 | - | - |
| | L-histidine (Ajinomoto Co., Inc.) | - | - | - | 440 | - | - |
| | Glycine (Ajinomoto Co., Inc.) | - | - | - | - | 1500 | - |
| | Hydroxypropyl cellulose (Nippon Soda Co., Ltd.) | - | 90 | 60 | 12 | 45 | - |
| | Partially pregelatinized starch (Asahi Kasei Chemicals Corporation) | - | 160 | - | - | - | - |
| | Purified water | - | 180 | 260 | 300 | 173 | - |
| | Ethanol | - | 60 | - | 100 | 58 | - |
| Coating step ingredients (g) | Uncoated granule ingredients | 300 | 300 | 300 | 300 | 300 | 300 |
| | Coating components | | | | | | |
| | (One-layer coating) | | | | | | |
| | Mannitol Mitsubishi Shoji Foodtech Co. Ltd.) | 30 | 30 | 30 | 30 | 30 | 30 |
| | Hydroxypropyl cellulose (Nippon Soda Co., Ltd.) | 18 | 18 | 18 | 18 | 18 | 18 |

### Test Example 4 Sensory Evaluation 2 of Taste

First, the uncoated granules shown in Table 6 were taken by 2 to 5 panelists to evaluate the taste. Then, corresponding coated granules were taken to perform a sensory evaluation on the taste having been evaluated on the uncoated granules. The evaluation criteria are as shown next. ○: indicates that the taste was not unpleasant at all, Δ: indicates that the taste was little unpleasant, and ×: indicates no change.

### [Table 7]

**Table 7**

| | Taste of uncoated granule | Sensory evaluation on coated granule |
|---|---|---|
| Coated granule of Example 14 | Strong bitterness/weak umami | Δ to ○ |
| Coated granule of Example 15 of | Umami | Δ to ○ |
| Coated granule of Example 16 | Weak umami/weak sweetness | Δ to ○ |
| Coated granule of Example 17 | Weak bitterness/ umami | Δ to ○ |
| Coated granule of Example 18 | Sweetness/cold feel | ○ |
| Coated granule of Example 19 | Bitterness/umami | Δ to ○ |

As shown in Table 7, the products of the present invention all had sufficiently satisfactory taste improvement.

Even with glutamic acid, glutamine, lysine, histidine, glycine, and threonine in addition to arginine, the products of the present invention had an improved taste, and furthermore excellent ease of ingestion was obtained with little aggregation in the oral cavity. Thus, the products of the present invention can be said to be a quite favorable preparation for the elderly who have reduced swallowing ability and having a small amount of saliva secreted.

### Test Example 5 Disintegration Test

According to the method described in the Japanese Pharmacopeia Fifteenth Edition, the disintegration test was performed on the coated granule preparation shown in Table 6. The evaluation criteria are as shown next. ○: immediate releasing is retained (the disintegration test is met), ×: immediate releasing is not retained (the disintegration test is not met).

### [Table 8]

**Table 8**

| | Immediate release |
|---|---|
| Coated granule of Example 14 | ○ |
| Coated granule of Example 15 | ○ |
| Coated granule of Example 16 | ○ |
| Coated granule of Example 17 | ○ |
| Coated granule of Example 18 | ○ |
| Coated granule of Example 19 | ○ |

As shown in Table 8, the products of the present invention all had the immediate releasing retained, and the disintegration failure did not occur due to the coating.

Using Robot Sifter (RPS-95C: Seishin Enterprise Co., Ltd.), the particle size distribution of the uncoated granules were determined. Table 9 shows the particle size distribution of the uncoated granules in Examples 1 to 10, 19, and Examples 14 to 18.

### [Table 9]

**Table 9**

| | | Uncoated granules of Examples 1 to 10, 19 | Uncoated granules of Example 14 | Uncoated granules of Example 15 | Uncoated granules of Example 16 | Uncoated granules of Example 17 | Uncoated granules of Example 18 |
|---|---|---|---|---|---|---|---|
| Particle size distribution (%) | 1400 µm on | 0.0 | 0.0 | 0.0 | 0.0 | 0.0 | 0.0 |
| | 1400 µm pass, 850 µm on | 0.8 | 1.6 | 4.9 | 1.2 | 0.7 | 5.0 |
| | 850 µm pass, 500 um on | 98.3 | 98.3 | 95.0 | 98.5 | 98.9 | 93.4 |
| | 500 µm pass, 355 µm on | 0.6 | 0.0 | 0.0 | 0.1 | 0.2 | 1.5 |
| | 355 µm pass | 0.3 | 0.1 | 0.1 | 0.1 | 0.1 | 0.1 |

## Claims

1. A hydrophilic amino acid-containing preparation **characterized in that**
a solid formulation containing a hydrophilic amino acid is coated with a coating agent containing a corrigent and a water-soluble polymer substance, and
the hydrophilic amino acid is in a form selected from the group consisting of a free form, a salt, a solvate, and mixtures thereof.

2. The hydrophilic amino acid-containing preparation according to claim 1, **characterized in that**
the hydrophilic amino acid is selected from the group consisting of arginine, arginine salts, and mixtures thereof.

3. The hydrophilic amino acid-containing preparation according to claim 1, **characterized in that**
the hydrophilic amino acid is selected from the group consisting of lysine, lysine salts, histidine, histidine salts, and mixtures thereof.

4. The hydrophilic amino acid-containing preparation according to any one of claims 1 to 3, **characterized in that**
the solid formulation containing the hydrophilic amino acid further contains, in addition to the hydrophilic amino acid, an amino acid other than the hydrophilic amino acid, and
the amino acid other than the hydrophilic amino acid is in a form selected from the group consisting of a free form, a salt, a solvate, and mixtures thereof.

5. The hydrophilic amino acid-containing preparation according to any one of claims 1 to 4, **characterized in that**
the corrigent is selected from the group consisting of aspartame, saccharin, sodium saccharin, glycyrrhizic acid, glycyrrhizates, acesulfame K, mannitol, erythritol, sorbitol, xylitol, trehalose, cacao powder, menthol, thaumatin, stevia, sucralose, maltitol, refined white sugar, white sugar, dextrose, fructose, and mixtures thereof.

6. The hydrophilic amino acid-containing preparation according to any one of claims 1 to 5, **characterized in that**
the water-soluble polymer substance is selected from the group consisting of hydroxypropyl cellulose, hydroxypropylmethyl cellulose, sodium carboxymethyl cellulose, methyl cellulose, gum arabic, polyvinylpyrrolidone (povidone), dextrin, sodium alginate, casein, and mixtures thereof.

7. The hydrophilic amino acid-containing preparation according to any one of claims 1 to 4, **characterized in that**
the corrigent is mannitol, and
the water-soluble polymer substance is hydroxypropyl cellulose.

8. The hydrophilic amino acid-containing preparation according to any one of claims 1 to 7, **characterized in that**
the coating agent further contains, in addition to the corrigent and the water-soluble polymer substance, one or more members selected from souring agents, plasticizers and lubricants.

9. The hydrophilic amino acid-containing preparation according to claim 8, **characterized in that**
the souring agent is selected from the group consisting of citric acid, malic acid, acetic acid, tartaric acid, ascorbic acid, glutamic acid, glutamates, lactic acid, succinic acid, maleic acid, malonic acid, and mixtures thereof.

10. The hydrophilic amino acid-containing preparation according to claim 8 or 9, **characterized in that**
the plasticizer is selected from the group consisting of macrogol, propylene glycol, triethyl citrate, castor oil, triacetin, polysorbate nonionic surfactants, sorbitan ester nonionic surfactants, and mixtures thereof.

11. The hydrophilic amino acid-containing preparation according to any one of claims 8 to 10, **characterized in that**
the lubricant is selected from the group consisting of talc, stearic acid, stearates, waxes, wheat starches, and mixtures thereof.

12. A solid preparation selected from a powder, a fine granule, a granule, a dry syrup, a pill, a tablet, a chewable, a capsule, and a microcapsule which are made of the hydrophilic amino acid-containing preparation according to any one of claims 1 to 11.

13. A hydrophilic amino acid-containing granule made of the hydrophilic amino acid-containing preparation according to any one of claims 1 to 11, the granule **characterized in that**
a ratio of the solid formulation containing the hydrophilic amino acid to the coating agent is 100/1 to 100/100 by weight.

14. A hydrophilic amino acid-containing granule made of the hydrophilic amino acid-containing preparation according to any one of claims 1 to 11, the granule **characterized in that**
the corrigent is mannitol,
the water-soluble polymer substance is hydroxypropyl cellulose, and
a ratio of the solid formulation containing the hydrophilic amino acid to the coating agent is 100/5 to 100/40 by weight.

15. A hydrophilic amino acid-containing tablet made of the hydrophilic amino acid-containing preparation according to any one of claims 1 to 11, the tablet **characterized in that**,
a ratio of the solid formulation containing the hydrophilic amino acid to the coating agent is 100/0.01 to 100/50 by weight.

16. A hydrophilic amino acid-containing tablet made of the hydrophilic amino acid-containing preparation according to any one of claims 1 to 11, the tablet further **characterized in that**
the corrigent is mannitol,
the water-soluble polymer substance is hydroxypropyl cellulose, and
a ratio of the solid formulation containing the hydrophilic amino acid to the coating agent is 100/0.1 to 100/20 by weight.
